# EUROPEAN PATENT SPECIFICATION

(11) **EP 4 506 027 B1**
(45) Date of publication and mention of the grant of the patent: **02.09.2026**
(21) Application number: 23784824.7
(22) Date of filing: 07.04.2023
(51) Int. Cl.: A61M 16/06, A61M 16/12, A61M 11/00, A61M 15/00, B65D 83/30, B65D 83/303, B65D 83/40

(54) **INHALATION MASK FOR AEROSOL CONTAINERS, AND AEROSOL PRODUCT COMPRISING SAID INHALATION MASK FOR AEROSOL CONTAINERS**
INHALATIONSMASKE FÜR AEROSOLBEHÄLTER UND AEROSOLPRODUKT MIT DIESER INHALATIONSMASKE FÜR AEROSOLBEHÄLTER
MASQUE D'INHALATION POUR RÉCIPIENTS D'AÉROSOL, ET PRODUIT D'AÉROSOL COMPRENANT LEDIT MASQUE D'INHALATION POUR RÉCIPIENTS D'AÉROSOL

(30) Priority: 08.04.2022 JP 2022064465
(43) Date of publication of application: 12.02.2025
(73) Proprietor: Mitani Valve Co., Ltd., Tokyo 103-0001 (JP)
(72) Inventor: TAKIZAWA Osamu, Tokyo 103-0001 (JP); HAZA Tatsuo, Tokyo 103-0001 (JP); WATANABE Tomonori, Tokyo 103-0001 (JP)
(74) Representative: SSM Sandmair
(86) International application number: PCT/JP2023/014452
(87) International publication number: WO 2023/195540

(56) References cited:
- EP-A2- 1 369 140
- JP-A- 2004 129 702
- JP-A- 2004 129 702
- JP-U- H0 184 656
- JP-U- H0 184 656
- JP-U- H0 480 880
- JP-U- S6 143 179
- JP-U- S6 143 179
- KR-B1- 102 097 147

## Description

### Technical Field

The present invention relates to an inhalation mask for an aerosol container, which is used attached to an aerosol type product.

In particular, the present invention relates to an inhalation mask for an aerosol container provided to cover an upper portion of the aerosol container as a cover cap during non-use.

Note here that there may be cases where the contents of the aerosol container in the aerosol type product is only gas, such as oxygen or nitrogen.

For mere convenience of explanation, the longitudinal direction of the stem, i.e., the up-and-down direction in each figure is referred to herein as "up" or "down"; a direction perpendicularly thereto is referred to as "horizontal"; a direction in which the contents are sprayed into an external space region, i.e., the upper left direction in Fig. 2 and the left direction in Figs. 3 and 4, is referred to as "front"; and a direction opposite thereto is referred to as "back".

### Background Art

Conventionally, various inhalation masks for aerosol type products have been proposed (refer to Patent Literatures 1 to 4).

These inhalation masks are configured to introduce not only the contents but also outside air for diluting the contents so that they can be sucked together without interfering with normal breathing.

Moreover, an injection of the contents is paused while the inhalation mask is placed over a mouth of a user, thereby allowing the user to suck only the introduced outside air.

An outside air introduction port for that purpose is provided on a rear surface of the inhalation mask or a bottom surface of the inhalation mask between an injection port of contents and the mouth of the user.

Even in Patent Literature 4, an outside air introduction port is not described in the [Front view diagram of the inhaler] in which a recess of a nose pad is provided, but is described in the [Rear view diagram of the inhaler], and therefore the outside air introduction port is provided at a portion which becomes a bottom surface during inhalation.

This is to keep a distance from an operation portion so that a user does not accidentally block the outside air introduction port during an operation.

These outside air introduction ports introduce outside air so as to be merged with the injected contents at the same time or in the middle of the injection, and therefore the contents and outside air are easily mixed with each other.

In particular, in inhalation masks respectively having small outside air introduction ports, the outside air flows into the contents with great force and they are mixed thoroughly.

Inhalation masks for aerosol containers are known from KR 102 097 147 B1, JP 2004 129702 A and JP H01 84656 U.

### Citation List

### Patent Literature

Patent Literature 1: Japanese Unexamined Utility Model Specification No. S61-043179 (JP-S61-043179-U1)
Patent Literature 2: Japanese Unexamined Utility Model Specification No. H01-084656 (JP-H01-084656-U1)
Patent Literature 3: Japanese Unexamined Utility Model Specification No. H01-084657 (JP-H01-084657-U1)
Patent Literature 4: Japanese design registration No. 1300855 (JP-1300855-S)

### Summary of Invention

### Technical Problem

However, while the contents and the outside air can be easily mixed with each other at these outside air introduction ports, the inevitable size of the inhalation mask covering the user's mouth causes the contents to diffuse into excess space inside the inhalation mask, resulting in not a little amount of wasted contents that remain uninhaled after use.

Moreover, the outside air is introduced so as to be merged with the injected contents at the same time or in the middle of the injection. Accordingly, the entire inhalation mask tends to be long and large, and when the aerosol container is attached, the inhalation mask may protrude sideways, thereby making it impossible to place the aerosol container on its own.

Therefore, an object of the present invention is to introduce outside air into an inhalation mask through an outside air introduction port provided on an operation surface so as not to be blocked by a user and to additionally introduce contents from an aerosol container, thereby allowing the user to actively suck the contents and making effective use of the contents.

Another object thereof is to miniaturize the inhalation mask by providing the outside air introduction port on the operation surface.

### Solution to Problem

The present invention solves the above-described problem by using the following configuration aspects.
(1) An inhalation mask (e.g., an inhalation mask 4 described below) for an aerosol container (e.g., an aerosol container 1 described below) is provided to cover an upper portion of the aerosol container during non-use, and includes an opening port (e.g., an opening port 4a described below) applied to a vicinity of a mouth of a user during use, a stem fitting portion (e.g., a stem fitting portion 4b described below) fitted to a stem (e.g., a stem 3 described below) of the aerosol container during use, a passage portion (e.g., a passage portion 4c described below) configured to introduce contents from the stem to an inside of the opening port, and a ventilation hole (e.g., a ventilation hole 4g described below) configured to introduce outside air to the inside of the opening port. The inhalation mask includes:
   an operation surface (e.g., an operation surface 4e described below) for depressing the stem; and
   an anti-slip recess (e.g., an anti-slip recess 4f described below) provided on the operation surface, wherein the ventilation hole is provided so as to be communicated with the anti-slip recess.
(2) The inhalation mask for the aerosol container according to the above-described configuration aspect (1), wherein
   a detachable extended portion (e.g., an extension pipe 5 described below) is provided downstream of the passage portion.
(3) The inhalation mask for the aerosol container according to the above-described configuration aspect (1) or (2), further includes
   an engagement portion (e.g., an inner cylindrical portion 4h and an engagement portion convex 4j described below) provided inside the opening port and detachably engaged with an upper portion of the aerosol container.
(4) The inhalation mask for the aerosol container according to any one of the above-described aspects (1) to (3), further includes
   a lower recess (e.g., a lower recess 4k described below) provided on an outer peripheral surface between the stem fitting portion and the opening port so as to face an outer peripheral portion of a mounting cup of the aerosol container during an injection operation, wherein
   the opening port is set 15 to 30 degrees upward with respect to an orthogonal direction of the stem that is fitted to the stem fitting portion.

The present invention provides an inhalation mask according to claim 1, and an aerosol type product according to claim 5. The dependent claims 2-4 define embodiments of the invention as claimed.

### Advantageous Effects of Invention

The present invention can make effective use of contents and miniaturize an inhalation mask by adopting the above-mentioned configuration.

### Brief Description of Drawings

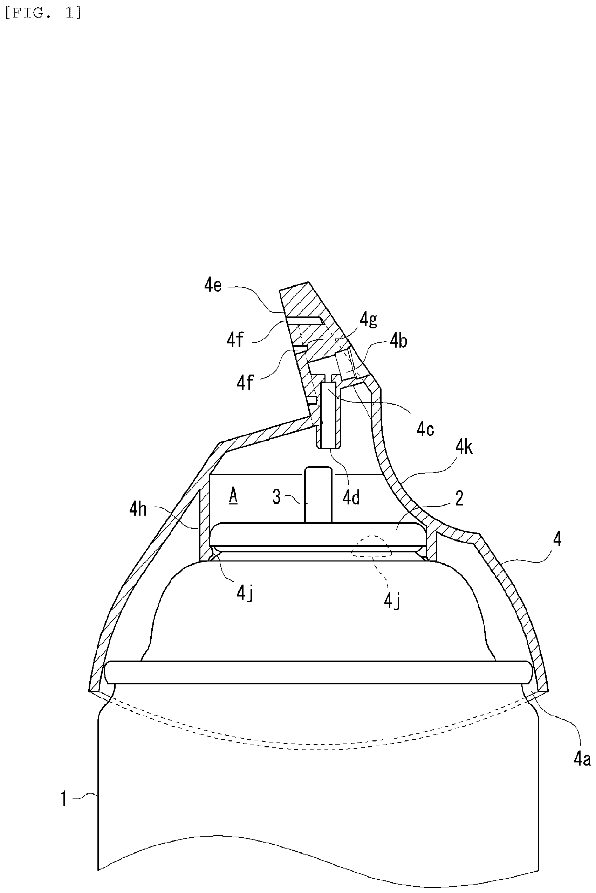

[Fig. 1] Fig. 1 is an explanatory diagram illustrating a non-use state of an inhalation mask 4 according to an embodiment of the invention as claimed.

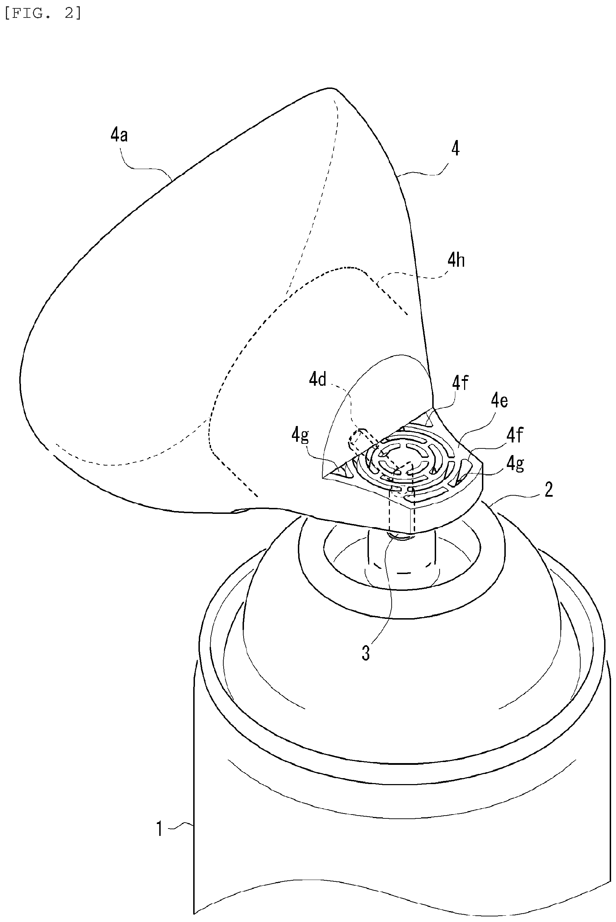

[Fig. 2] Fig. 2 is an explanatory diagram illustrating a use state of the inhalation mask 4 illustrated in Fig. 1.

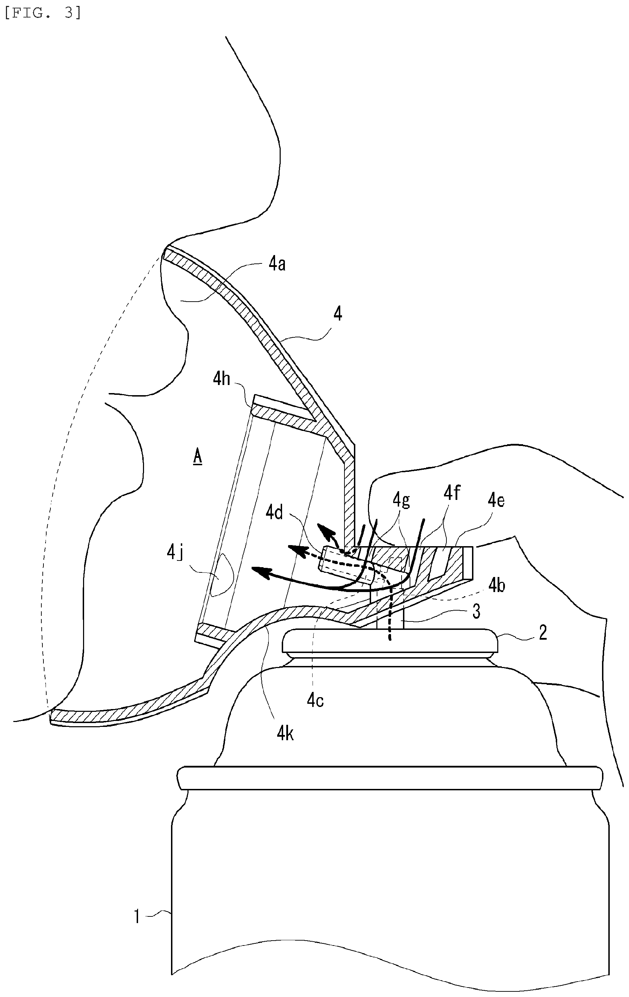

[Fig. 3] Fig. 3 is an explanatory diagram illustrating an injection state of the inhalation mask 4 illustrated in Fig. 1.

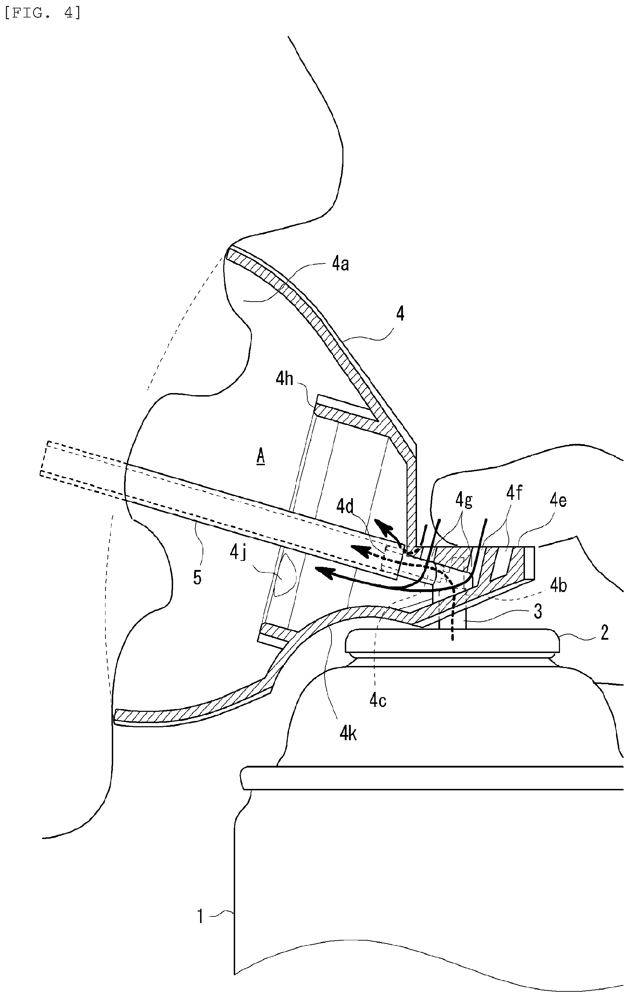

[Fig. 4] Fig. 4 is an explanatory diagram illustrating an injection state when attaching an extension nozzle 5 to the inhalation mask 4 illustrated in Fig. 1.

### Description of Embodiments

Embodiments of the present invention will be described with reference to Figs. 1 to 4.

In principle, in the following description, a component denoted by a reference sign including an alphabetical suffix (e.g., an operation surface 4e) indicates a part of a component denoted by a numeral part of the reference sign (e.g., an inhalation mask 4).

As illustrated in Figs. 1 to 4,
reference sign 1 denotes an aerosol container accommodating contents to be injected;
reference sign 2 denotes a mounting cup attached to an upper opening port of the aerosol container 1;
reference sign 3 denotes a cylindrical stem provided so as to pass through a center opening port of the mounting cup 2 and releases the contents of the aerosol container 1 from an upper-end hole portion by a depressing operation;
reference sign 4 denotes a cup-shaped inhalation mask, according to an embodiment of the invention as claimed, applied to a periphery of a mouth of a user to prevent diffusion of released contents;
reference sign 4a denotes an open-side end portion of the cup-shaped inhalation mask 4, which is an opening port applied to the periphery of the mouth of the user;
reference sign 4b denotes a stem fitting portion formed on a close-side peripheral surface of the cup-shaped inhalation mask 4 and fitted to an upper end of the stem;
reference sign 4c denotes a passage portion communicated from the stem fitting portion 4b to an inside of the inhalation mask 4;
reference sign 4d denotes a downstream end portion of the passage portion 4c, which is a release port through which the contents are injected;
reference sign 4e denotes a close-side peripheral surface of the cup-shaped inhalation mask 4, which is an operation surface formed on an opposite side of the stem fitting portion 4b and subjected to the depressing operation by the user;
reference sign 4f denotes an anti-slip recess formed of a plurality of grooves provided on the operation surface 4e;
reference sign 4g denotes a ventilation hole communicated from the anti-slip recess 4f to the inside of the inhalation mask 4;
reference sign 4h denotes an inner cylindrical portion engaged with an outer peripheral surface of the mounting cup 2;
reference sign 4j denotes an engagement portion engaged with a lower side of the outer peripheral surface of the mounting cup 2;
reference sign 4k denotes a lower recess provided so as to face the outer peripheral portion of the mounting cup 2 during use to prevent the inhalation mask 4 from interfering with the mounting cup 2 during an injection operation;
reference sign 5 denotes an extension pipe detachably provided on an outer periphery of the release port 4d to set an injecting position of the contents; and
reference sign A denotes an internal space region of the cup-shaped inhalation mask 4.

Herein, the stem 3, the inhalation mask 4, and the extension pipe 5 are formed of, for example, plastics such as polypropylene, polyethylene, polyacetal, nylon, or polybutylene terephthalate.

The aerosol container 1 is formed of, for example, plastic or metal, and the mounting cup 2 is formed of, for example, metal.

Note here that in a central lower portion of the mounting cup 2, there are provided a stem gasket forming a release valve together with the stem 3, a stem spring biasing the stem 3 upwards and holds this release valve in the closed state, and a housing holding these members with a lower end side of the stem 3 (not illustrated), which are well known.

As an example, the contents to be filled in the aerosol container 1 are gas only, such as oxygen or gaseous helium mixed with oxygen.

Fig. 1 illustrates an aerosol type product according to an embodiment of the invention as claimed during non-use in a state where the inhalation mask 4 is provided to cover an upper portion of the aerosol container 1.

The inhalation mask 4 is in a state where the opening port 4a faces downward and the upper portion of the aerosol container 1 is inserted into the opening port 4a. An outer periphery of the mounting cup 2 is fitted to an inner periphery of the inner cylindrical portion 4h, and the engagement portion 4j is locked to a lower end of the outer periphery of the mounting cup 2, thereby preventing the inhalation mask 4 from unintended falling away.

There is an annular recessed portion on an upper surface of the mounting cup 2 around the stem 3 which easily accumulate foreign matters such as dust, but this is prevented by covering the entire upper portion of the aerosol container 1 with the inhalation mask 4.

Since the aerosol type product in such a state during non-use is mainly stored upright, the stem fitting portion 4b and the ventilation hole(s) 4g are in a state of being opened in a lateral direction, making it difficult for foreign matters such as dust to enter the internal space region A through these portions.

Moreover, the stem 3 is also protected by the inhalation mask 4, thereby preventing the contents from being released even when an external force is applied to the aerosol type product in such a state.

Fig. 2 illustrates the aerosol type product in a use state in which the inhalation mask 4 is removed from the mounting cup 2 of the aerosol container 1 and then is attached to the stem 3 from the state illustrated in Fig. 1.

The stem 3 and the stem fitting portion 4b are airtightly and liquid-tightly fitted to each other so that the inhalation mask 4 can be attached and detached without easily falling away.

In a state where the aerosol container 1 illustrated in Fig. 2 is upright, the opening port 4a is set slightly upward from the horizontal, and the operation surface 4e is set horizontally.

Since the inhalation mask 4 is directly attached to the stem 3, and the operation surface 4e provided with the ventilation hole 4g is disposed directly above the stem 3, no space for providing an outside air introduction port is necessary, thereby reducing a horizontal protrusion of the inhalation mask 4 with respect to the aerosol container 1 and miniaturizing the inhalation mask 4.

Therefore, even when the aerosol container 1 is placed on a horizontal surface in this state, it does not topple due to a weight of the inhalation mask 4.

Fig. 3 illustrates the aerosol type product in an injection state in which a user grips the aerosol type product in the use state illustrated in Fig. 2 and the inhalation mask 4 is applied to a mouth to depress the operation surface 4e.

In this state, when a user sucks, the inside of the inhalation mask 4 becomes negative pressure, outside air flows into the internal space region A through the ventilation hole(s) 4g in the anti-slip recess(es) 4f, and on the way, the contents of the aerosol container 1 are merged through the depressed stem 3, the passage portion 4c, and the release port 4d to be sucked.

Since user's fingers cannot completely close all of the anti-slip recesses 4f, it can ensure an inflow of outside air through the ventilation hole(s) 4g.

Since the outside air from the ventilation hole 4g flows into the internal space region A and then the contents are merged with the outside air at the place near the mouth of the user, both are sucked by the user without being violently mixed.

A proportion of the contents remaining in the internal space region A of the inhalation mask 4 after the inhalation is completed is reduced, and the contents that are not sucked by the user and are diffused in an external space region and wasted are reduced, resulting in effective use of the contents.

Since the lower recess 4k prevents the inhalation mask 4 from coming into contact with the outer peripheral portion of the mounting cup 2 during the injection operation, a position of the inhalation mask 4 with respect to the aerosol container 1 can be moved near the aerosol container 1 side, thereby reducing a horizontal protrusion of the inhalation mask 4 with respect to the aerosol container 1.

Consequently, it becomes difficult for the aerosol container 1 to topple due to the weight of the inhalation mask 4 when the aerosol container 1 is placed, and also enables the user to easily apply the inhalation mask 4 to the own mouth without moving the own hand holding the aerosol container 1 away from the own face.

Moreover, since the opening port 4a is set 15 degrees upward from the horizontal direction, the upper portion of the opening port 4a in the inhalation mask 4 is closer to the aerosol container 1 side and less likely to topple, and it also becomes unnecessary for the user to raise a position of the own hand gripping the aerosol container 1 to a height of the own mouth.

The upward angle of the opening port 4a for attaching the inhalation mask 4 to the stem 3 of the aerosol container 1 can be within a range from 0 degree to 90 degrees, but is preferably within a range from 15 degrees to 30 degrees.

Within this range of angles, the lower recess 4k can be provided in a size that does not affect an action of the engagement portion 4j and the like, and the operation surface 4e can easily be disposed directly above the stem 3 as described above.

Furthermore, reducing the horizontal protrusion of the inhalation mask 4 to shorten an overall length thereof also contributes to a reduction in a height and miniaturization of the aerosol type product in the non-use state illustrated in Fig. 1.

Fig. 4 illustrates the aerosol type product in an injection state in a case of attaching an extension pipe 5 to the ventilation hole 4g. The configuration except for the extension pipe 5 is the same as that illustrated in Fig. 3.

Since the contents are released near the mouth of the user through the extension pipe 5, the amount of the contents that is not sucked and is diffused into the external space region and is wasted is reduced, and the contents are effectively utilized.

The length of the extension pipe 5 is appropriately selected in accordance with the type of contents. For example, if a physical problem occurs when a user holds the extension pipe 5 in own mouth and sucks only the contents, the extension pipe can be made short so as not to reach the user's mouth in order to ensure that outside air is also sucked.

In some cases, the contents may be mixed with oxygen gas in advance so that the user does not experience physical problems even if the user sucks only the contents.

After use, the inhalation mask 4 is removed upward from the stem 3 in the state illustrated in Figs. 2 to 4, the extension pipe 5, if any, is also be removed from the release port 4d, and the upper portion of the aerosol container 1 can be covered with the opening port 4a of the inhalation mask 4 to be stored in the state illustrated in Fig. 1.

Naturally, the present invention is not limited to the above-described embodiments but may be modified as follows:
(11) a spray nozzle structure is provided on the release port 4d;
(12) the extension pipe 5 is made to have an extendable bellows shape instead of a detachable cylindrical member;
(13) the inside and outside of the extension pipe 5 are made permeable with microscopic hole portions communicating with each other to prevent the user from sucking only the contents even when the user holds the extension pipe 5 in own mouth;
(14) a groove for passing outside air is provided on an outer peripheral surface of the extension pipe 5 to prevent the user from sucking only the contents even when the user holds the extension pipe 5 in own mouth;
(15) the extension pipe 5 is made to be extendable in accordance with a pressure and a flow force of the contents passing through; and/or
(16) a spraying tip for atomizing the contents is provided on the release port 4d.

The aerosol type products to which the present invention is applied include various applications, such as inhaled oxygen agents, inhaled air agents, humectants, voice changing agents, throat humidifying agents, inhaled pharmaceuticals, and flavoring agents.

The contents contained in the aerosol container that may be used include various forms such as gas, liquid materials, powdered materials, and the like. The components that may be contained in the contents include, for example, oxygen gas, nitrogen gas, helium gas, components effective for individual applications, water, and the like.

The powdered materials that may be used include glucose, sucrose, salt, mixtures thereof, and the like.

The oil components that may be used include, for example, ester oil such as isopropyl myristate, oils and fats such as palm oil, eucalyptus oil, camellia oil, olive oil, and the jojoba oil, a fatty acid such as myristic acid, palmitic acid, stearic acid, linoleic acid, linolenic acid, and the like.

The alcohols that may be used include, for example, monohydric lower alcohols such as ethanol, monohydric higher alcohols such as lauryl alcohol and cetanol, and polyalcohols such as ethylene glycol, 1,3-butylene glycol, glycerin, and the like.

The surfactants that may be used include, for example, an anionic surfactant such as sodium lauryl sulfate, a nonionic surfactant such as polyoxyethylene alkyl ether and polyglycerin fatty acid ester, an amphoteric surfactant such as lauryl dimethyl aminoacetic acid betaine, and a cationic surfactant such as alkyl trimethyl ammonium chloride, and the like.

The high molecular compounds that may be used include, for example, hydroxyethyl cellulose, methyl cellulose, gelatin, starch, casein, xanthan gum, carboxyvinyl polymer, and the like.

The components effective for individual applications that may be used include, for example, vitamins such as retinol and dl-α-tocopherol, humectants such as hyaluronic acid, antiphlogistic balms such as methyl salicylate and indomethacin, sodium benzoate, refreshments such as camphor and menthol, antiasthmatic drugs such as ephedrine and adrenalin, sweeteners such as sucralose or aspartame, and the like.

Furthermore, it is possible to use, besides the above-described contents, for example, suspending agents, emulsifying agents, antioxidants, and sequestering agents, and the like.

Injection gas (propellants) in the aerosol type products that may be used include, compressed gas such as compressed gas, such as oxygen gas, compressed air, nitrogen gas, carbon dioxide gas, rare gas, and mixed gases thereof, liquefied gases such as liquefied petroleum gas, dimethyl ether, fluorocarbon, and the like.

### Reference Signs List

1: Aerosol container
2: Mounting cup
3: Stem
4: Inhalation mask
4a: Opening port
4b: Stem fitting portion
4c: Passage portion
4d: Release port
4e: Operation surface
4f: Anti-slip recess
4g: Ventilation hole
4h: Inner cylindrical portion
4j: Engagement portion
4k: Lower recess
5: Extension pipe
A: Internal space region

## Claims

1. An inhalation mask (4) for an aerosol container (1) provided tc cover an upper portion of the aerosol container during non-use, the inhalation mask including an opening port (4a) applied to a vicinity of a mouth of a user during use, a stem fitting portion (4b) fitted to a stem (3) of the aerosol container during use, a passage portion (4c) configured to introduce contents from the stem to an inside of the opening port, and a ventilation hole (4g) configured to introduce outside air to the inside of the opening port, the inhalation mask comprising:
an operation surface (4e) for depressing the stem; and **characterised in that** the inhalation mask further comprises:
an anti-slip recess (4f) provided on the operation surface, and **in that**
the ventilation hole is provided so as to be communicated with the anti-slip recess.

2. The inhalation mask (4) according to claim 1, wherein
a detachable extended portion (5) is provided downstream of the passage portion (4c).

3. The inhalation mask (4) according to claim 1 or 2, further comprising
an engagement portion (4j) provided inside the opening port (4a) and detachably engaged with an upper portion of the aerosol container (1).

4. The inhalation mask (4) according to claim 1 or 2, further comprising
a lower recess (4k) provided on an outer peripheral surface between the stem fitting portion (4b) and the opening port (4a) so as tc face an outer peripheral portion of a mounting cup (2) of the aerosol container (1) during an injection operation, wherein
the opening port is set 15 to 30 degrees upward with respect to an orthogonal direction of the stem (3) that is fitted to the stem fitting portion.

5. An aerosol type product comprising
the inhalation mask (4) according to claim 1 or 2, wherein the aerosol type product accommodates the contents.

## Patentansprüche

1. Inhalationsmaske (4) für Aerosolbehälter (1), die dazu vorgesehen ist, während des Nichtgebrauchs einen oberen Bereich des Aerosolbehälters abzudecken, wobei die Inhalationsmaske umfasst:
eine Öffnung (4a), die während des Gebrauchs an die Umgebung des Mundes eines Benutzers angelegt wird;
einen Schaftaufnahmeabschnitt (4b), der während des Gebrauchs auf einen Schaft (3) des Aerosolbehälters aufgesetzt wird;
einen Durchgangsabschnitt (4c), der dazu eingerichtet ist, den Inhalt vom Schaft in das Innere der Öffnung einzuleiten;
eine Belüftungsöffnung (4g), die dazu eingerichtet ist, Außenluft in das Innere der Öffnung einzuleiten; die Inhalationsmaske umfassend:
eine Betätigungsfläche (4e) zum Niederdrücken des Schafts;
**dadurch gekennzeichnet, dass** die Inhalationsmaske ferner
eine auf der Betätigungsfläche vorgesehene rutschhemmende Vertiefung (4f) umfasst,
und dass die Belüftungsöffnung so vorgesehen ist, dass sie mit der rutschhemmenden Vertiefung in Verbindung steht.

2. Inhalationsmaske (4) für Aerosolbehälter nach Anspruch 1,
wobei stromabwärts des Durchgangsabschnitts (4c) ein abnehmbarer Verlängerungsabschnitt (5) vorgesehen ist.

3. Inhalationsmaske (4) für Aerosolbehälter nach Anspruch 1 oder 2,
ferner umfassend:
einen Eingriffsabschnitt (4j), der innerhalb der Öffnung (4a) vorgesehen ist und abnehmbar mit einem oberen Bereich des Aerosolbehälters (1) in Eingriff steht.

4. Inhalationsmaske (4) für Aerosolbehälter nach Anspruch 1 oder 2,
ferner umfassend:
eine untere Vertiefung (4k), die auf einer Außenumfangsfläche zwischen dem Schaftaufnahmeabschnitt (4b) und der Öffnung (4a) vorgesehen ist, sodass sie während eines Ausstoßvorgangs einem Außenumfangsabschnitt eines Montagebechers (2) des Aerosolbehälters (1) gegenüberliegt,
wobei die Öffnung um 15 bis 30 Grad nach oben gegenüber einer orthogonalen Richtung des Schafts (3), der in den Schaftaufnahmeabschnitt eingesetzt ist, eingestellt ist.

5. Aerosolprodukt, umfassend die Inhalationsmaske (4) für Aerosolbehälter nach Anspruch 1 oder 2,
wobei das Aerosolprodukt den Inhalt enthält.

## Revendications

1. Masque d'inhalation (4), pour un contenant d'aérosol (1) prévu pour couvrir une partie supérieure du contenant d'aérosol durant la non-utilisation, le masque d'inhalation incluant un orifice d'ouverture (4a) appliqué sur un voisinage d'une bouche d'un utilisateur durant l'utilisation, une partie d'ajustement de tige (4b) ajustée sur une tige (3) du contenant d'aérosol durant l'utilisation, une partie de passage (4c) configurée pour introduire un contenu, depuis la tige, à l'intérieur de l'orifice d'ouverture, et un trou de ventilation (4g) configuré pour introduire de l'air extérieur à l'intérieur de l'orifice d'ouverture, le masque d'inhalation comprenant :
une surface d'opération (4e) pour abaisser la tige ; et
**caractérisé en ce que** le masque d'inhalation comprend en outre :
un évidement anti-glissement (4f) prévu sur la surface d'opération, et **en ce que** le trou de ventilation est prévu afin d'être mis en communication avec l'évidement anti-glissement.

2. Masque d'inhalation (4) selon la revendication 1, dans lequel
une partie étendue détachable (5) est prévue en aval de la partie de passage (4c).

3. Masque d'inhalation (4) selon la revendication 1 ou 2, comprenant en outre
une partie d'entrée en prise (4j) prévue à l'intérieur de l'orifice d'ouverture (4a) et en prise de façon détachable avec une partie supérieure du contenant d'aérosol (1).

4. Masque d'inhalation (4) selon la revendication 1 ou 2, comprenant en outre
un évidement inférieur (4k) prévu sur une surface périphérique extérieure entre la partie d'ajustement de tige (4b) et l'orifice d'ouverture (4a) afin de faire face à une partie périphérique extérieure d'une coupelle de montage (2) du contenant d'aérosol (1) durant une opération d'injection, dans lequel
l'orifice d'ouverture est réglé à 15 à 30 degrés vers le haut par rapport à une direction orthogonale de la tige (3) qui est ajustée sur la partie d'ajustement de tige.

5. Produit de type aérosol, comprenant le masque d'inhalation (4) selon la revendication 1 ou 2, dans lequel le produit de type aérosol loge le contenu.
